# EUROPEAN PATENT SPECIFICATION

(11) **EP 3 796 955 B1**
(45) Date of publication and mention of the grant of the patent: **25.10.2023**
(21) Application number: 19752399.6
(22) Date of filing: 31.07.2019
(51) Int. Cl.: A61M 1/16

(54) **SORBENT CARTRIDGE FOR DIALYSATE REGENERATION**
SORPTIONSKARTUSCHE ZUR REGENERIERUNG VOM VERBRAUCHTEN DIALYSAT
CARTOUCHE ADSORBANTE DE RÉGÉNÉRATION DU DIALYSAT

(30) Priority: 31.07.2018 US 201862712674 P
(43) Date of publication of application: 31.03.2021
(73) Proprietor: Fresenius Medical Care Holdings, Inc., Waltham, MA 02451-1457 (US)
(72) Inventor: ADAMS, Kerissa, Waltham, Massachusetts 02451-1457 (US); MOSS, Jon, Waltham, Massachusetts 02451-1457 (US); JENSEN, Lynn, Waltham, Massachusetts 02451-1457 (US); MERCHANT, Stephen, Waltham, Massachusetts 02451-1457 (US)
(74) Representative: Fish & Richardson P.C.
(86) International application number: PCT/US2019/044306
(87) International publication number: WO 2020/028473

(56) References cited:
- WO-A2-02/43859
- US-A1- 2003 098 270
- US-A1- 2017 189 598

## Description

### TECHNICAL FIELD

This invention relates to sorbent cartridge devices for regenerating dialysis solution.

### BACKGROUND

Renal dysfunction or failure and, in particular, end-stage renal disease, causes the body to lose the ability to remove water and minerals and excrete harmful metabolites, maintain acid-base balance and control electrolyte and mineral concentrations within physiological ranges. Toxic uremic waste metabolites, including urea, creatinine, and uric acid, accumulate in the body's tissues which can result in a person's death if the filtration function of the kidney is not replaced.

Dialysis is commonly used to replace kidney function by removing these waste toxins and excess water. In one type of dialysis treatment--hemodialysis--toxins are filtered from a patient's blood externally in a hemodialysis machine. Blood passes from the patient through a dialyzer separated by a semi-permeable membrane from a large volume of externally-supplied dialysis solution. The waste and toxins dialyze out of the blood through the semi-permeable membrane into the dialysis solution, which is then discarded.

Hemodialysis treatments are typically conducted at a clinic since the hemodialysis machines generally require a continuous water source, reverse osmosis machinery, and drain lines for discarding the large volumes of water and dialysis solution used during a single treatment. Hemodialysis treatment typically must be performed three or four times a week, under supervision of the clinical staff, requirements that significantly decrease a patient's autonomy and quality of life.

Certain devices reconstitute used dialysis solution from hemodialysis and/or peritoneal dialysis as opposed to discarding it. The dialysis solution can be regenerated in a machine employing a device that eliminates urea from the solution.
US 2017/0189598 describes sorbent cartridge systems that are useful in regenerating or purifying dialysis solutions are described as well as methods to regenerate or purify spent dialysis solutions. Dialysis systems using the sorbent cartridge system are also described. WO 02/43859 describes cartridges that are used in regenerating or purifying dialysis solutions as well as methods to regenerate or purify spent dialysis solutions. Dialysis systems using the sorbent cartridges are further described.
US 2003/098270 describes filter cartridge assemblies and housings that include a tubular housing having an inner wall, an outer wall, a first end, and a second end. The housings include inner walls with shoulders or other radially-inwardly extending flow directors at the intersections of adjacent sections of the tubular body. The assemblies include a plurality of filter media sections within the housing, and each of the plurality of filter media sections preferably has a different filter media composition. One or more of the filter media sections traverses one or more of the shoulders or other radially-inwardly extending flow directors such that the flow directors evenly direct the flow of fluid through the assembly. The assemblies find particular applicability in dialysis systems.

### SUMMARY

This disclosure generally relates to dialysis systems.

The sorbent-based dialysis systems typically include a module that is capable of regenerating dialysis solution (e.g., dialysate). The module typically includes a sorbent device for filtering used or spent dialysis solution so that the used solution can be recycled for repeated use during a dialysis treatment. By locating the fluid access points of the sorbent device at one end of the sorbent device, the sorbent device can be made easier to use when users attach fluid lines, and easier to place and store the sorbent device.

In embodiments, a sorbent cartridge device includes a housing defining a housing interior, a fluid coupling attached to the housing and configured to fluidically connect at least one fluid line to the housing interior, a baffle attached to a bottom of the housing interior and fluidically connected to the housing interior, the baffle configured to direct fluid flow entering the baffle radially and circumferentially, and a fluid accumulation module attached at the top of the housing and fluidically connected to the housing interior, the fluid accumulation module configured to direct fluid flow entering the fluid accumulation module from a bottom surface of the fluid accumulation module towards a center port of the module.

In some instances, the sorbent cartridge device can include one or more of the following. The fluid coupling is attached to the housing at a top surface of the housing. The fluid coupling includes a fluid inlet channel and a fluid outlet channel. A straw fluidically connected to the fluid inlet channel, the straw spanning a height of the housing and terminating at the baffle. The fluid coupling is attached to a side of the housing near a top surface of the housing. A second fluid coupling attached to a side of the baffle. The housing interior comprises a replaceable sorbent cartridge. The housing interior is filled with ion exchange materials. The baffle is removable from the housing.

In some embodiments, a dialysis system includes a dialysate generation machine, a pump adapted to move fluid through the dialysate generation machine, and a sorbent cartridge device fluidically connected to the dialysate generation machine, the device comprising a housing defining a housing interior, a fluid coupling attached to the housing and configured to fluidically connect at least one fluid line to the housing interior, a cylindrical baffle attached to a bottom of the housing interior and fluidically connected to the housing interior, the baffle configured to direct fluid flow entering the baffle radially and circumferentially, and a fluid accumulation module attached at the top of the housing and fluidically connected to the housing interior, the fluid accumulation module configured to direct fluid flow entering the fluid accumulation module from a bottom surface of the fluid accumulation module towards a center port of the module.

In some instances, the system can include one or more of the following. The fluid coupling is attached to the housing at a top surface of the housing. The fluid coupling includes a fluid inlet channel and a fluid outlet channel. A straw fluidically connected to the fluid inlet channel, the straw spanning a height of the housing and terminating at the baffle. The fluid coupling is attached to a side of the housing near a top surface of the housing. A second fluid coupling attached to a side of the baffle. The housing interior comprises a replaceable sorbent cartridge. The housing interior is filled with ion exchange materials. The baffle is removable from the housing.

Advantages of the systems and devices described herein include ease of use for the user. A single-port access simplifies attachment when a user is preparing to use the sorbent device for a dialysate treatment. This may be particularly advantageous for home dialysis users, who may not be medical professionals accustomed to making such connections. Another advantage is that by relocating the access ports for fluid to enter and exit the cartridge, the bottom of the canister can be made flat. Many known sorbent cartridges have a bottom access connection that cannot be flattened or disrupted. To use such a sorbent device, the device must be attached so that it is suspended from an associated dialysate machine, or a "skirt" must be built around the bottom of the device to accommodate the bottom port. No such accommodation is required for the sorbent devices described herein, simplifying the machine, as well as making it more cost effective and compact.

The details of one or more embodiments of the invention are set forth in the accompanying drawings and the description below. Other aspects, features, and advantages of the invention will be apparent from the description and drawings. The invention is defined by the appended claims.

### DESCRIPTION OF DRAWINGS

FIG 1A is a schematic of a dialysis system that includes a dialysate generation machine and a module with a sorbent cartridge device.
FIG 1B is a schematic of filtration layers that can be used with the sorbent cartridge device of FIG. 1A.
FIGS. 2A and 2B are perspective and cut-away views of a sorbent cartridge device for use in the system of FIG 1A.
FIG 3A is a cut-away view of the sorbent cartridge device of FIG 2B showing additional features.
FIG 3B is an exploded assembly view of the sorbent cartridge device of FIG 2B.
FIGS. 4A and 4B are cross-sectional views of components of the sorbent cartridge device of FIG 2B.
FIGS. 5A and 5B are close up and cross-sectional views of components of the sorbent cartridge device of FIG. 2B.
FIG 6 is a perspective view of a second sorbent cartridge device for use in the system of FIG. 1A.
FIGS. 7A and 7B are cut-away and exploded views of the sorbent cartridge device of FIG. 6.

### DETAILED DESCRIPTION

This disclosure generally relates to dialysis systems.

The sorbent-based dialysis systems typically include a module that is capable of regenerating dialysis solution (e.g., dialysate). The module typically includes a sorbent device for filtering used or spent dialysis solution so that the used solution can be recycled for repeated use during a dialysis treatment. By locating the fluid access points of the sorbent device at one end of the sorbent device, the sorbent device can be made easier to use when users attach fluid lines, and easier to place and store the sorbent device.

This disclosure generally relates to dialysis systems and methods. The dialysis systems typically include a module that is capable of regenerating dialysis solution (e.g., dialysate). The module typically includes a sorbent device for filtering used or spent dialysis solution so that the used solution can be recycled for repeated use during a dialysis treatment. By relocating the fluid access points of the sorbent device, the sorbent device can be made easier to use when users attach fluid lines, and easier to place and store.

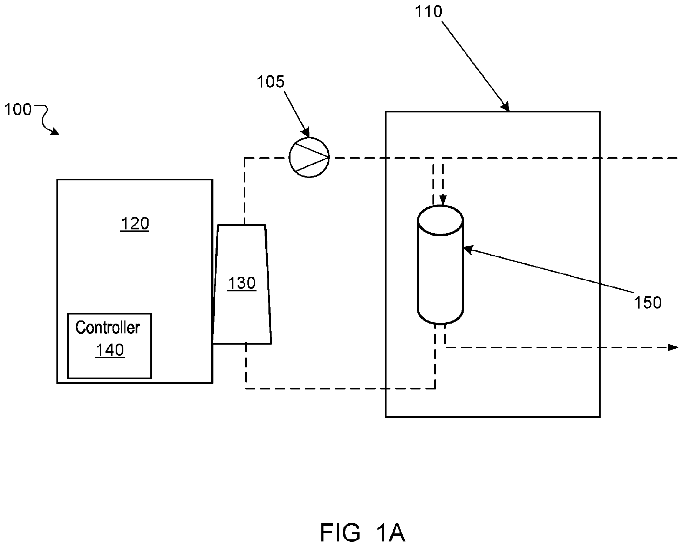

FIG 1A shows a schematic dialysis system 100 in which spent dialysis solution is moved under the force of at least one pump 105 from a dialysate generation machine 110 into a dialysate module 120 where it passes through a sorbent device 130 for recycling. The recycled dialysis solution exiting the sorbent device 130 is moved back to the dialysate generation machine 110. As the spent dialysis solution is passed through the sorbent device 130, toxins, such as urea, and other substances, such as calcium, magnesium, sodium, and potassium are stripped from the spent dialysis solution. To compensate for these materials being stripped from the dialysis solution, calcium, magnesium, potassium, and sodium levels of the recycled dialysis solution can be altered (e.g., by introducing calcium, magnesium, potassium, sodium, and/or a diluent into the dialysis solution) to restore concentrations of those substances to desired levels. A controller 140 controls the functions of the dialysate module 120.

As the recycled dialysis solution then passes through a dialyzer 150 connected to or associated with the dialysate generation machine 110, toxins are transferred from the patient's blood into the dialysis solution, forming spent dialysis solution. This spent dialysis solution is then circulated through the dialysis module 120 with the sorbent device 130 again to recycle or regenerate the spent dialysis solution. This process can be repeated until a desired amount of toxins have been removed from the patient's blood. Because the dialysis solution is recycled during the treatment as opposed to simply being discarded, the volume of dialysis solution used during the treatment can be substantially reduced relative to certain conventional hemodialysis techniques. In addition, maintaining the concentration of the various substances within the dialysis solution, such as calcium, magnesium, potassium, and sodium, can help to prevent the patient from experiencing discomfort during the treatment.

The sorbent device 130 includes a housing containing a sorbent cartridge capable of removing uremic toxins. In some embodiments, the cartridge is disposable. The cartridge can, for example, be constructed such that it can be removed from the housing after use and disposed of. The cartridge could then be replaced with a similar cartridge for a subsequent use of the dialysate module 120. The cartridge can purify water and regenerate spent dialysis solution through the use of a series of layers which can remove heavy metals (e.g., lead, mercury, arsenic, cadmium, chromium and thallium), oxidants (e.g., chlorine and chloramine), urea, phosphate and other uremic waste metabolites (e.g., creatinine and uric acid) from the solution.

In some embodiments, the components of the cartridge that perform the aforementioned functions include a purification layer that includes activated carbon; an ion exchange layer that includes a polymer phosphate binder or an ion exchange sorbent; and a urea removal layer that includes strong acid cation exchange resin and basic resin(s) or urea-degrading enzymes and an ion exchange sorbent together with a composition that rejects cations (e.g., flat membrane / hollow fibers, an ion-exchange membrane, or an encapsulation surrounding the urea removal components).

In certain embodiments, the cartridge includes the following layers and materials: sodium zirconium carbonate or other alkali metal-Group IV metal-carbonate; zirconium phosphate or other ammonia adsorbents; alumina or other like material; alumina supported urease or other immobilized enzyme layer or other material to convert urea to ammonia, such as diatomaceous earth or zirconium oxide; and granular activated carbon, such as charcoal, or other adsorbent. The sodium zirconium carbonate component can act as a phosphate adsorbent. The zirconium oxide can be capable of acting as a counter ion or ion exchanger to remove phosphate, and can be in the form of hydrous zirconium oxide (e.g., hydrous zirconium oxide containing acetate). The zirconium oxide can also be blended with the sodium zirconium carbonate when positioned in the cartridge.

Non-limiting examples of urea-degrading enzymes that can be employed in the sorbent cartridge include enzymes that are naturally occurring (e.g. urease from jack beans, other seeds or bacteria), produced by recombinant technology (e.g., in bacterial, fungal, insect or mammalian cells that express and/or secrete urea-degrading enzymes) or produced synthetically (e.g., synthesized). In some embodiments, the enzyme is urease.

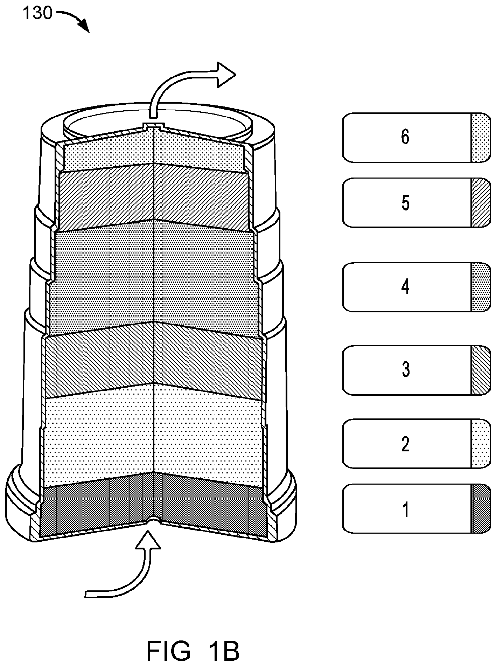

FIG 1B shows an example of the layers and materials that may be used within the housing of the sorbent device 130. The sorbent device includes six layers of materials, which are designed to remove contaminants and uremic solutes while at the same time maintaining an appropriate dialysate composition. Spent dialysate flows through the cartridge from bottom to top. The first and third layers with which the dialysate comes into contact contain activated carbon. These layers adsorb heavy metals, chloramines, and other contaminants that can be found in the tap water. In addition, the activated carbon adsorbs many of the organic and middle molecule uremic solutes found in spent dialysate, including creatinine and uric acid. The second layer is an enzyme-retention layer. The enzyme present is urease, which catalyzes the conversion of urea to ammonium bicarbonate. The fourth layer contains zirconium phosphate and is a cation exchange layer. Its primary function is to adsorb the ammonium ion generated by urea hydrolysis that took place in the second layer. In addition, this cation exchange material adsorbs other positively charged species such as magnesium, calcium, and potassium, as well as heavy metal cations that may be found in tap water such as copper and iron. In exchange for the adsorbed cations, the zirconium phosphate releases hydrogen and sodium. The fifth layer is an anion exchange layer containing zirconium oxide. This material adsorbs phosphate, fluoride, and other anions, such as oxoanions of heavy metals, and in exchange release chloride and hydroxyl anions. The sixth layer contains sodium bicarbonate. It does not bind anything but releases sodium and bicarbonate.

In certain embodiments, the sorbent cartridge further includes hollow fibers. The hollow fibers can reject positively charged ions, as well as increase the capacity of the cartridge. The hollow fibers can be coated with an ion-rejecting material, which through a water-purification like mechanism allows the urea through but rejects positively charged ions such as calcium and magnesium. The material coating the hollow fibers can be any such material known to one of skill in the art (e.g., fatty acids or polymer chains like polysulfone) that can effectively reject calcium and magnesium and therefore retain the ions in the dialysis solution. Generally, to have this effect the material itself would be positively charged. In some embodiments, for example, the material used to coat the hollow fibers is cellulose acetate (e.g., cellulose triacetate). The hollow fibers that are to be coated are commercially available (e.g., Fresenius Medical Care North America) and can be coated with any desired ion-rejecting material available to one having skill in the art.

Alternatively, the hollow fibers can include an ion-selective nanofiltration membrane. Such membranes are commercially available from a number of sources (e.g., Amerida, Koch, GE, Hoechst and Dow Chemical). These membranes have pores sizes that prevent ionic substances from diffusing through the membrane. For example, there are nanofiltration membranes that have an ability to reject ions with more than one negative charge (e.g., sulfate and phosphate) while allowing single-charged ions to pass through, with the converse also being the case. In either case, the hollow fiber devices are available in a variety of dimensions and need only be small enough to fit in the replaceable cartridge, which can be sized for use in an in-home system.

In certain embodiments, the sorbent cartridge can further include a flat membrane that is covered with a positively charged material like those described above. In addition, the membrane can be an ion exchange (e.g., anion) membrane that limits the passage of positively charged ions. Advantageously, this ion exchange membrane also has an ability to adsorb phosphate.

The cartridge and/or its components or layers can be replaced (e.g., membrane, urea-degrading enzyme), regenerated (e.g., resin, sorbent) and/or sterilized for re-use when necessary (e.g., saturation, damage, depletion). In addition, the entire cartridge can be replaceable and thus removed from the dialysis system when there is a decrease in the regeneration efficiency of the cartridge (e.g., through layer saturation) or the cartridge becomes worn or damaged, for instance.

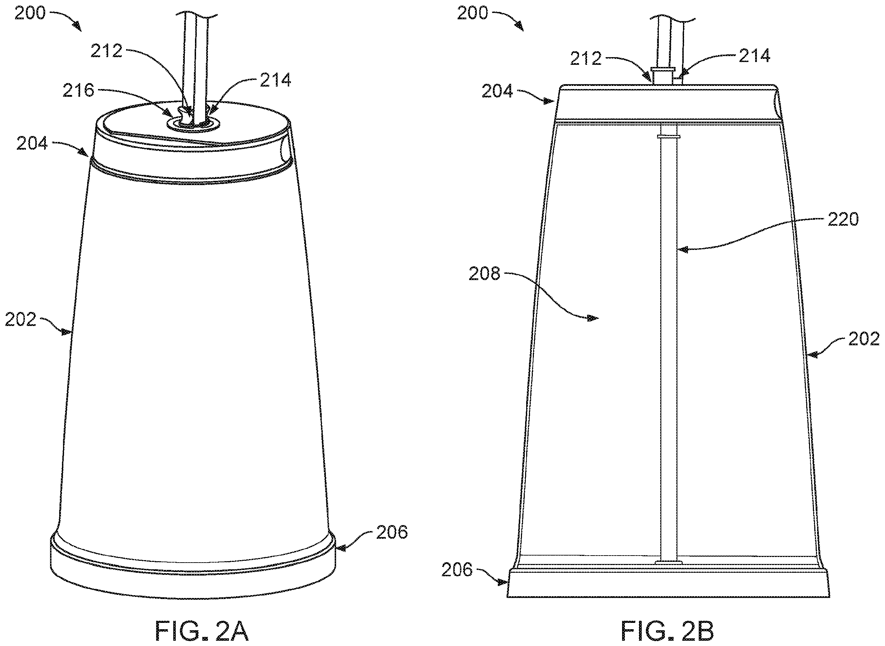

FIGS. 2A and 2B show a sorbent device 200 for use with the dialysate module of 120 of FIG 1A (e.g., the sorbent device 130) where the fluid access is solely through a top portion of the device. As can be seen by the exterior view of FIG. 2A and the cut-away view of FIG. 2B, the sorbent device 200 includes a housing 202 having a housing top 204 and a housing bottom 206. As can be seen in FIG 2B, the housing bottom 206 is generally flat, allowing the sorbent device 200 to rest on a flat surface. The housing top 204 may be integral with the housing 202. The housing 202 can be made of any suitable material, e.g., medical-grade plastic. The housing can be disposable or reusable and formed of a material that can be withstand either chemical or heat sterilization.

Within the housing 202 is an interior cavity 208 that holds the filter elements, such as a sorbent cartridge capable of removing uremic toxins. The filter elements are not shown for clarity, but typically fill the interior cavity 208 and are formed with any of the materials and features discussed above. In some embodiments, the cartridge and housing 202 can be constructed such that the cartridge can be removed from the housing 202 and disposed of after use, e.g., by removing the housing bottom 206. The cartridge could then be replaced with a similar cartridge for a subsequent use of the dialysate module 120.

The sorbent device 200 is a single-connection device. Rather than the bottom inlet and top outlet of typical sorbent devices, the housing top 204 includes both the fluid inlet 212 and fluid outlet 214 at a fluid access 216. The fluid inlet 212 and fluid outlet 214 are fluidically connected to dialysate tubing that allows both spent and recycled dialysate to flow to the dialyzer 150 (in FIG 1A).

Referring to FIG 2B, a fluid straw 220 extends down through the interior cavity 208 from the fluid access 216 in the housing top 202 to within the housing bottom 206.

In use, fluid (e.g., spent dialysate) flows to the sorbent device 200 from the dialyzer 150 to the fluid inlet 212, and through the fluid straw 220 to the bottom of the sorbent housing 202. The fluid exits the fluid straw 220 at the bottom of the housing 202 and then rises around the outside of the fluid straw 202, and is filtered through the filter elements that fill the interior cavity 208. The fluid diffuses through the various sorbent layers filling the interior cavity 208, leaving various substances within the filter layer, until the filtered fluid rises to the top portion of the housing 202. The filtered fluid is collected within the housing top 204 and directed to the fluid exit 214. The fluid then leaves the sorbent device 200 close to where it entered via the fluid inlet 212 and is infused with electrolytes and directed to the dialyzer 150 and the patient undergoing treatment.

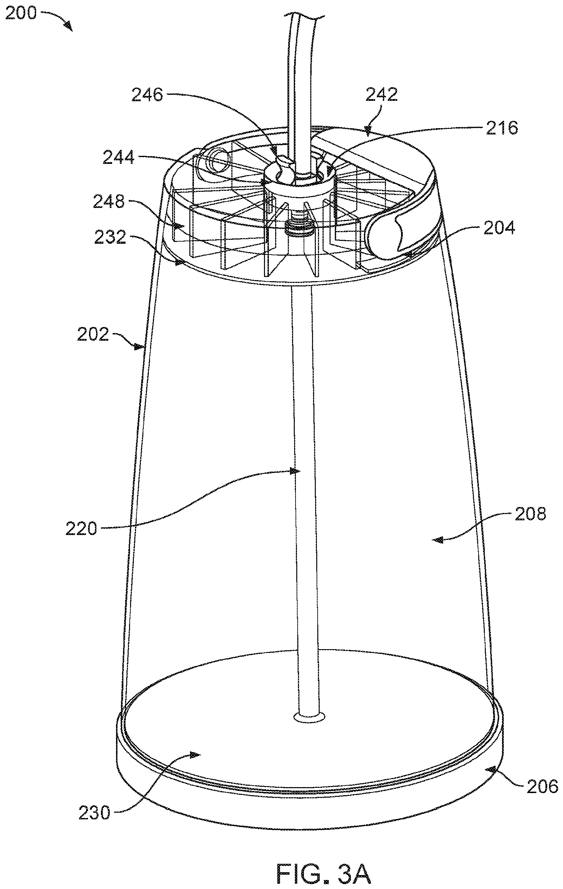

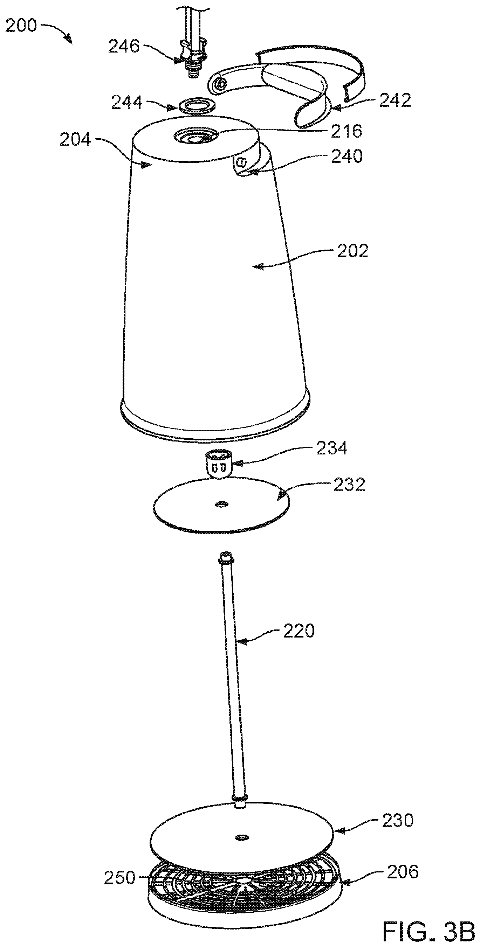

FIGS. 3A and 3B show further details of the sorbent device 200. The housing bottom 206 can include a particulate filter 230 that is bonded to the casing of the housing bottom 206 and the housing top 204 also includes a particulate filer 232. The housing bottom 206 is detachable from the rest of the housing 202 as shown in FIG 3B. The fluid straw 220 is attached to the housing top 204 using a straw canister adapter 234. The housing top 204 includes a depression 240 that allows a handle 242 to fit snugly against the housing 202 when the handle 242 is not in use. The handle 242 is rotatable from its stored position to an upright, in-use position. The fluid access 216 includes a hole through the housing 204 in which a latch ring 244 fits. The latch ring 244 can be pressed in, snapped in, or bonded to the housing 202. The latch ring 244 mates with a coupling 246 that connects fluid tubing to the fluid straw 220. As seen in FIG 3A, a fluid accumulation module 248 fits into the housing 202 at the housing top 204 while as seen in FIG. 3B, a baffle 250 is included in the housing bottom 206.

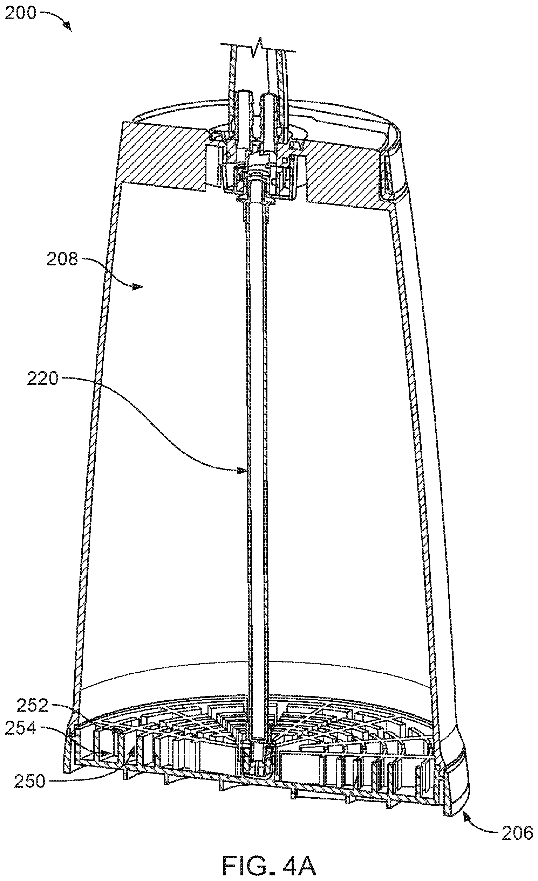

The baffle 250 is generally cylindrical. Portions of the baffle 250 are also visible in FIG. 4A. These portions include multiple channels 252 defined by multiple separators 254. As can be seen in both FIG. 4A and FIG. 3B, the channels 252 and separators 254 of the baffle 250 are arranged in a radial pattern symmetrically centered on the outlet of the fluid straw 220. This radial pattern disperses the fluid exiting the fluid straw 220 circumferentially evenly around the circular bottom of the sorbent device 200. The baffle 250 permits fluid flow through the channels 252, flow upwards out of the baffle 250, but not flow downwards.

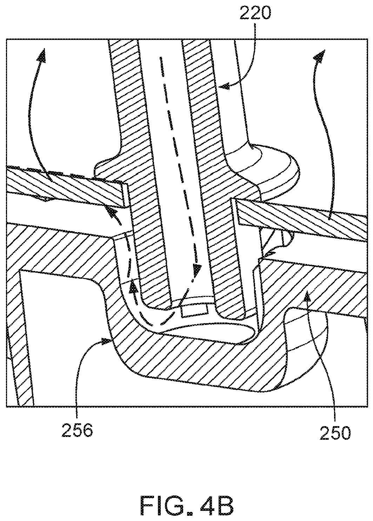

The arrangement of the baffle 250 includes a central divot 256 that holds the fluid straw 220 firmly in the center of the baffle 250 while fluid is exiting the straw 220 and flowing upward through the filtering elements (as illustrated in FIG 4B). The central positioning of the straw 220, and the arrangement of the baffle 250, ensures that fluid flow exiting the straw 220 is smoothed and evenly dispersed around the housing bottom 206. The even dispersal of the exiting fluid ensures that the fluid progresses evenly up through the sorbent layers within the interior cavity 208, allowing all surface area of the filter layers to participate in the fluid filtration.

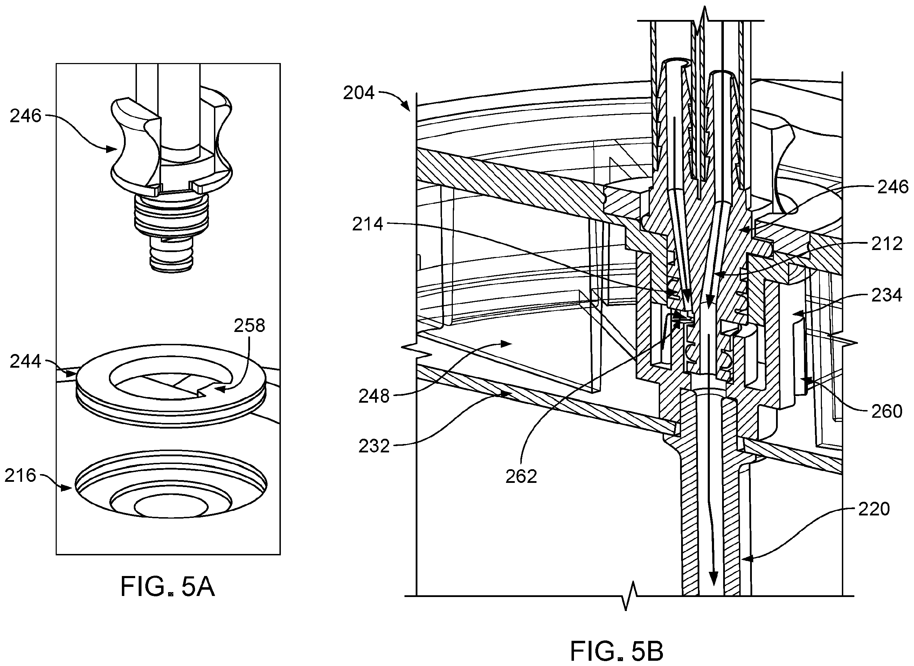

FIGS. 5A and 5B show elements of the housing top 204. The coupling 246 connects fluid tubing to the interior cavity 208 so that the fluid access 216 (e.g., including the fluid inlet 212 and fluid exit 214) is centrally positioned on the top surface of the sorbent device 200. This central position is ensured by a locking mechanism, such as the latch ring 244 that includes a locking notch 258. The coupling 246 can be a standard fluid coupling such as a PTC22020 coupling insert, from Colder Products Company^{®}.

When inserted into the housing top 204, the coupling 246 fluidically connects the fluid inlet 212 with the top of the fluid straw 220. Fluid travelling in through the fluid inlet 212 is directed down through the length of the fluid straw 220, where it exits. As shown in FIG. 4B, as it exits, the fluid is evenly directed outwards from the center line of the sorbent device 200 by the baffle 250 in the housing bottom 206, such that the fluid is circumferentially evenly distributed around the fluid straw 220. Under pressure from the pump 105 driving the fluid through the fluid inlet 212, the fluid then percolates upwards through the filtering elements of the interior cavity 208.

When the filtered fluid reaches the housing top 204, it travels through the final filter layer of the particulate filter 232 that separates the interior cavity 208 from the fluid accumulation module 248 at the housing top 204. As can be seen in FIGS. 3A and 5B, the fluid accumulation module 248 has a radial arrangement somewhat similar to the radial arrangement of the baffle 250 at the bottom of the housing. The radial arrangement of the fluid accumulation module directs fluid bubbling up along the longitudinal axis of the sorbent device 200 (e.g., parallel to the axis of the fluid straw 220 and roughly parallel to the walls of the housing 202) to move radially inwards towards the centerline of the sorbent device 200. The fluid impinges the straw canister adapter 234, which attaches the coupling 246 to the fluid accumulation module 248. The straw canister adapter 234 includes multiple access ports 260 radially arranged around its circumference. The access ports 260 fluidically connect the fluid accumulation module 248 with the coupling 248. A single exit port 262 connected to fluid exit 214 allows fluid entering the straw canister adapter 234 via the access ports 260 to be directed back into the coupling 246 and pumped out of the sorbent device 200 along a fluid tube. Alternatively, there could be multiple points to collect the cleaned solution, while maintaining a separation between the inlet and outlet.

The fluid inlet 212 and fluid outlet 214 are both located on the top surface of housing top 204. In the illustrated embodiment, only the fluid inlet 212 is positioned in the centerline of the sorbent device 200. The fluid outlet 214 is positioned slightly off-center. In some embodiments, the fluid outlet 214 can be positioned far off-center and distant from the fluid inlet 212. In this instance, more than one coupling might join the fluid tubing to the sorbent device 200. In some embodiments, the fluid inlet 212 can be positioned far off-center of the fluid housing 202. In this instance the baffle 250 is configured to direct the flow so that the spent dialysate percolates through the layers evenly without channeling.

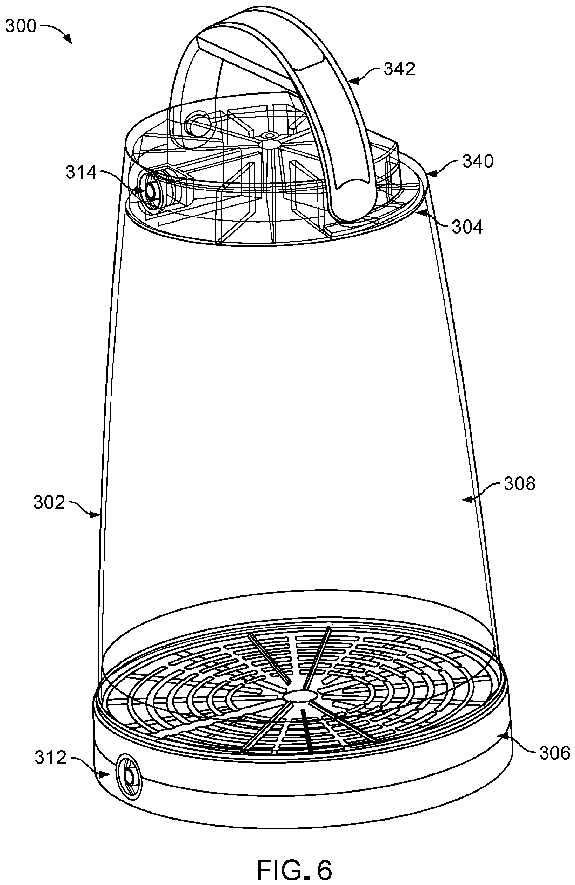

FIG 6 shows another embodiment of a sorbent device 300 for use with the dialysate module of 120 of FIG 1A (e.g., the sorbent device 130) where the fluid access is through a top portion as well as a bottom portion of the device. The sorbent device 300 includes a housing 302 with a housing top 304 and a housing bottom 306. The housing bottom 306 is generally flat, allowing the sorbent device 300 to rest on a flat surface.

Within the housing 302 is an interior cavity 308 that holds the filter elements such as a sorbent cartridge capable of removing uremic toxins, which are not shown for clarity. The cartridge and housing 3020 can be constructed such that the cartridge can be removed from the housing 302 and disposed of after use, e.g., by removing the housing bottom 306. The cartridge could then be replaced with a similar cartridge for a subsequent use of the dialysate module 120.

Unlike the single-connect sorbent device 200 described above, the sorbent device 300 is a dual-connection device. The dual-connection sorbent device 300 has a bottom-placed fluid inlet 312 in the housing bottom 306 and a fluid outlet 314 at the housing top 304. The fluid inlet 312 and fluid outlet 314 are each located on a side wall of the housing 302, rather than the top surface as in sorbent device 200, or on the top and bottom surfaces of prior sorbent devices. The fluid inlet 312 and fluid outlet 314 are each fluidically connected to dialysate tubing that allows both spent and recycled dialysate to flow to the dialyzer 150 (in FIG. 1A). The fluid inlet 312 and fluid outlet 314 can include any type of fluid coupling, such as luer connectors or DIN connectors. The housing top 304 includes a depression 340 that allows a handle 342 to fit snugly against the housing 302.

In use, fluid (e.g., spent dialysate) flows to the sorbent device 300 from the dialyzer 150 to the fluid inlet 312 and then rises and is filtered through the filter elements that fill the interior cavity 308. The fluid diffuses through the various sorbent layers filling the interior cavity 308, leaving various substances within the filter layer, until the filtered fluid rises to the top portion of the housing 302. The filtered fluid is collected within the housing top 304 and directed to the fluid exit 314 at the side of the housing top 304. The fluid then leaves the sorbent device 300 and is infused with electrolytes and directed to the dialyzer 150 and the patient undergoing treatment.

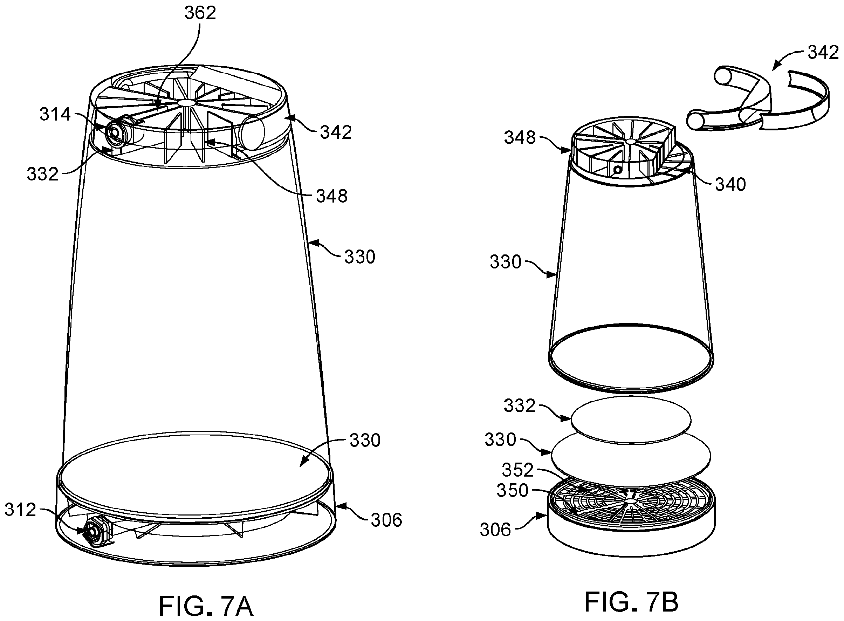

FIGS. 7A and 7B show further details of the sorbent device 300. The housing bottom 306 includes a particulate filter 330 that can be bonded to the casing of the housing bottom 306 and the housing top 304 also includes a particulate filer 332. The housing bottom 306 is detachable from the rest of the housing 302 as shown in FIG 7B. A fluid accumulation module 348 fits into the housing 302 at the housing top 304 while, as seen in FIG 7B, a baffle 350 is included in the housing bottom 306.

Portions of the baffle 350 are also visible in FIG 7A. Similar to sorbent device 200, the baffle 350 includes multiple channels defined by multiple separators. These channels include an inlet channel 352, which directs fluid entering the housing bottom 304 via the fluid inlet 312 towards the centerline of the sorbent device 300. The channels and separators of the baffle 350 are arranged in a radial pattern symmetrically centered on the centerline of the sorbent device. This radial pattern disperses the fluid exiting the inlet channel 352 circumferentially evenly around the circular bottom of the sorbent device 300 before it flows percolates through the filtering elements. The arrangement of the baffle 350 ensures that fluid flow entering the interior cavity 308 is smoothed and evenly dispersed 306. The even dispersal of the exiting fluid ensures that the fluid progresses evenly up through the sorbent layers without channeling within the interior cavity 308, allowing all surface area of the filter layers to participate in the fluid filtration. Under pressure from the pump 105 driving the fluid through the fluid inlet 312, the fluid then percolates upwards through the filtering elements of the interior cavity 308.

When the filtered fluid reaches the housing top 304, it travels through the final filter layer of the particulate filter 332 that separates the interior cavity 308 from the fluid accumulation module 348 at the housing top 304. The fluid accumulation module 348 has a radial arrangement somewhat similar to the radial arrangement of the baffle 350 at the bottom of the housing. The radial arrangement of the fluid accumulation module directs fluid bubbling up along the longitudinal axis of the sorbent device 300 (e.g., roughly parallel to the walls of the housing 302) to move radially inwards towards the centerline of the sorbent device 300. The fluid is collected at the center and directed out of the sorbent device 300 via an outlet channel 362 that terminates in the fluid exit 314, where it is pumped out of the sorbent device 300 along a suitable fluid tube.

A number of embodiments of the invention have been described. Nevertheless, it will be understood that various modifications may be made without departing from the spirit and scope of the invention.

For example, in some embodiments, the features of sorbent device 200 and sorbent device 300 can be combined. In this instance, the straw seen in sorbent device 200 may not go through the center of the cavity interior. Instead, the straw is attached to the outside of the housing and runs from the top to the bottom of the housing. A single-access port similar to that of sorbent device 200 allows a single connection to the top of the cartridge. Rather than directing the fluid into the housing at the top, this cartridge's coupling redirects the incoming fluid along the outside of the housing, and allows the fluid to enter the cartridge through a side port as in sorbent device 300. A similar baffle arrangement as in sorbent device 300 would redistribute the fluid to progress evenly up through the filler layers within the interior.

In some instances, the housing bottom can include two layers, including a top layer that houses a baffle, as well as a bottom layer. The bottom layer can include a fluid inlet that is an extension of the straw that is attached to the outside of the housing. This fluid inlet directs the incoming fluid to the centerline of the cartridge, where is then distributed radially and circumferentially around the bottom of the cartridge.

In some instances, the fluid may enter the top and be pumped with the aid of gravity to the bottom of the cartridge where a fluid exit is located.

While the sorbent modules have been described as being used with a hemodialysis system, they can be used with other types of blood treatment systems that use dialysate, including hemodiafiltration systems and peritoneal dialysis systems.

## Claims

1. A sorbent cartridge device for regenerating dialysis solution (200, 300) comprising
a housing (202, 302) defining a housing interior (208, 308);
a fluid coupling (212, 214, 312, 314) attached to the housing and configured to fluidically connect at least one fluid line to the housing interior;
a baffle (250, 350) attached to a bottom of the housing interior and fluidically connected to the housing interior, the baffle configured to direct fluid flow entering the baffle radially and circumferentially; and
a fluid accumulation module (248, 348) attached at a top of the housing and fluidically connected to the housing interior, the fluid accumulation module configured to direct fluid flow entering the fluid accumulation module from a bottom surface of the fluid accumulation module towards a center port of the module.

2. The device of claim 1, wherein the fluid coupling is attached to the housing at a top surface of the housing.

3. The device of claim 2, wherein the fluid coupling includes a fluid inlet channel and a fluid outlet channel.

4. The device of claim 3, comprising a straw (220) fluidically connected to the fluid inlet channel, the straw spanning a height of the housing and terminating at the baffle.

5. The device of claim 1, wherein the fluid coupling is attached to a side of the housing near a top surface of the housing.

6. The device of claim 5, comprising a second fluid coupling attached to a side of the baffle.

7. The device of any of the previous claims, wherein the housing interior comprises a replaceable sorbent cartridge.

8. The device of any of the previous claims, wherein the housing interior is filled with ion exchange materials.

9. The device of any of the previous claims, wherein the baffle is removable from the housing.

10. A dialysis system (100) comprising:
a dialysate generation machine (110);
a pump (105) adapted to move fluid through the dialysate generation machine; and
a sorbent cartridge device according to any preceding claim fluidically connected to the dialysate generation machine.

## Patentansprüche

1. Sorbenskartuschenvorrichtung zum Regenerieren von Dialyselösung (200, 300), umfassend:
ein Gehäuse (202, 302), das einen Gehäuseinnenraum (208, 308) definiert;
eine Fluidkopplung (212, 214, 312, 314), die an dem Gehäuse angebracht ist und dafür gestaltet ist, wenigstens eine Fluidleitung mit dem Gehäuseinnenraum in Fluidverbindung zu bringen;
eine Umlenkplatte (250, 350), die an einem Boden des Gehäuseinnenraums angebracht ist und in Fluidverbindung mit dem Gehäuseinnenraum steht, wobei die Umlenkplatte dafür gestaltet ist, Fluidstrom, der zu der Umlenkplatte einritt, radial und umfangsseitig zu lenken; und
ein Fluidakkumulationsmodul (248, 348), das an einer Oberseite des Gehäuses angebracht ist und in Fluidverbindung mit dem Gehäuseinneren steht, wobei das Fluidakkumulationsmodul dafür gestaltet ist, Fluidstrom, der in das Fluidakkumulationsmodul eintritt, von einer unteren Oberfläche des Fluidakkumulationsmoduls in Richtung zu einer mittleren Öffnung des Moduls zu lenken.

2. Vorrichtung gemäß Anspruch 1, wobei die Fluidkopplung an einer oberen Oberfläche des Gehäuses an dem Gehäuse angebracht ist.

3. Vorrichtung gemäß Anspruch 2, wobei die Fluidkopplung einen Fluideinlasskanal und einen Fluidauslasskanal enthält.

4. Vorrichtung gemäß Anspruch 3, umfassend einen Halm (220) in Fluidverbindung mit dem Fluideinlasskanal, wobei der Halm eine Höhe des Gehäuses überspannt und an der Umlenkplatte endet.

5. Vorrichtung gemäß Anspruch 1, wobei die Fluidkopplung an einer Seite des Gehäuses nahe einer oberen Oberfläche des Gehäuses angebracht ist.

6. Vorrichtung gemäß Anspruch 5, umfassend eine zweite Fluidkopplung, die an einer Seite der Umlenkplatte angebracht ist.

7. Vorrichtung gemäß einem der vorstehenden Ansprüche, wobei der Gehäuseinnenraum eine ersetzbare Sorbenskartusche umfasst.

8. Vorrichtung gemäß einem der vorstehenden Ansprüche, wobei der Gehäuseinnenraum mit Ionenaustauschmaterialien gefüllt ist.

9. Vorrichtung gemäß einem der vorstehenden Ansprüche, wobei die Umlenkplatte von dem Gehäuse entfernbar ist.

10. Dialysesystem (100), umfassend:
eine Dialysaterzeugungsmaschine (110);
eine Pumpe (105), die dafür ausgelegt ist, Fluid durch die Dialysaterzeugungsmaschine zu bewegen; und
eine Sorbenskartuschenvorrichtung gemäß einem der vorstehenden Ansprüche in Fluidverbindung mit der Dialysaterzeugungsmaschine.

## Revendications

1. Dispositif de cartouche de sortant pour régénérer une solution de dialyse (200, 300) comprenant :
un boîtier (202, 302) définissant un intérieur de boîtier (208, 308) ;
un raccord de fluide (212, 14, 312, 314) fixé au boîtier et configuré pour relier fluidiquement au moins une ligne de fluide à l'intérieur de boîtier ;
un déflecteur (250, 350) fixé à un partie inférieure de l'intérieur de boîtier et raccordé fluidiquement à l'intérieur de boîtier, le déflecteur étant configuré pour diriger l'écoulement de fluide entrant dans le déflecteur radialement et circonférentiellement ; et
un module d'accumulation de fluide (248, 348) fixé au niveau d'une partie supérieure du boîtier et raccordé fluidiquement à l'intérieur de boîtier, le module d'accumulation de fluide étant configuré pour diriger l'écoulement de fluide entrant dans le module d'accumulation de fluide à partir d'une surface inférieure du module d'accumulation de fluide vers un orifice central du module.

2. Dispositif selon la revendication 1, le raccord de fluide étant fixé au boîtier sur une surface supérieure du boîtier.

3. Dispositif selon la revendication 2, le raccord de fluide comprenant un canal d'entrée de fluide et un canal de sortie de fluide.

4. Dispositif selon la revendication 3, comprenant une paille (220) raccordée fluidiquement au canal d'entrée de fluide, la paille s'étendant sur une hauteur du boîtier et se terminant au niveau du déflecteur.

5. Dispositif selon la revendication 1, le raccord de fluide étant fixé sur un côté du boîtier à proximité d'une surface supérieure du boîtier.

6. Dispositif selon la revendication 5, comprenant un second raccord de fluide fixé sur un côté du déflecteur.

7. Dispositif selon l'une quelconque des revendications précédentes, l'intérieur de boîtier comprenant une cartouche de sorbant remplaçable.

8. Dispositif selon l'une quelconque des revendications précédentes, l'intérieur de boîtier étant rempli de matériaux échangeurs d'ions.

9. Dispositif selon l'une quelconque des revendications précédentes, le déflecteur pouvant être retiré du boîtier.

10. Système de dialyse (100) comprenant :
une machine de génération de dialysat (110) ;
une pompe (105) adaptée pour déplacer le fluide à travers la machine de génération de dialysat ; et
un dispositif de cartouche de sortant selon l'une quelconque des revendications précédentes raccordé fluidiquement à la machine de génération de dialysat.
